# EUROPÄISCHE PATENTSCHRIFT

(11) **EP 0 823 890 B1**
(45) Veröffentlichungstag und Bekanntmachung des Hinweises auf die Patenterteilung: **08.12.1999**
(21) Anmeldenummer: 96914977.2
(22) Anmeldetag: 23.04.1996
(51) Int. Cl.: C07C 69/734, C07C 67/343, C07C 67/327, C07C 67/333

(54) **VERFAHREN UND ZWISCHENPRODUKTE ZUR HERSTELLUNG VON IM WESENTLICHEN ISOMERENREINEN E-2-[2-ARYLOXYMETHYLENPHENYL]-CROTONSÄUREMETHYLESTERN**
PROCESS AND INTERMEDIATE PRODUCTS FOR PREPARING SUBSTANTIALLY ISOMER-PURE E-2-[2-ARYLOXYMETHYLENE PHENYL]-CROTONIC ACID METHYL ESTERS
PROCEDE ET PRODUITS INTERMEDIAIRES DE PREPARATION D'ISOMERES SENSIBLEMENT PURS D'ESTERS METHYLIQUES D'ACIDE E-2-[2-ARYLOXYMETHYLENE PHENYLE]-CROTONIQUE

(30) Priorität: 04.05.1995 DE 19516407
(43) Veröffentlichungstag der Anmeldung: 18.02.1998
(73) Patentinhaber: BASF AKTIENGESELLSCHAFT, 67056 Ludwigshafen (DE)
(72) Erfinder: GRAMMENOS, Wassilios, D-67063 Ludwigshafen (DE); GÖTZ, Norbert, D-67547 Worms (DE); SAUTER, Hubert, D-68167 Mannheim (DE); OBERDORF, Klaus, D-69117 Heidelberg (DE)
(86) Internationale Anmeldenummer: EP9601689
(87) Internationale Veröffentlichungsnummer: WO9634847

(56) Entgegenhaltungen:
- EP-A- 0 280 185

## Beschreibung

Die vorliegende Erfindung betrifft ein Verfahren zur Herstellung von im wesentlichen isomerenreinem E-2-[2-Aryloxymethylenphenyl]-crotonsäuremethylestern der Formel Ia, 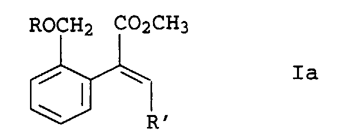 in der
- R: für Phenyl oder Heteroaryl, welches partiell oder vollständig halogeniert sein kann und/oder
- einen bis drei der folgenden Gruppen tragen kann: Cyano, Nitro, C₁-C₄-Alkyl, C₁-C₄-Halogenalkyl, C₁-C₄-Alkyloxy, C₁-C₄-Halogenalkyloxy, C₁-C₄-Alkylthio, C₁-C₄-Halogenalkylthio, C₃-C₈-Cycloalkyl, Phenyl, Phenoxy oder Benzyloxy, wobei die cyclischen Reste ihrerseits partiell oder vollständig halogeniert sein können und/oder einen bis drei der folgenden Gruppen tragen kann: Cyano, Nitro, C₁-C₄-Alkyl, C₁-C₄-Halogenalkyl, C₁-C₄-Alkyloxy, C₁-C₄-Halogenalkyloxy, C₁-C₄-Alkylthio, C₁-C₄-Halogenalkylthio, C₃-C₈-Cycloalkyl, Phenyl, Phenoxy oder Benzyloxy, und/oder
- eine CRⁱ=NORⁱⁱ-Gruppe tragen kann, in der
   Rⁱ Wasserstoff, Cyano, Halogen,
   C₁-C₆-Alkyl, C₁-C₆-Alkoxy, C₁-C₆-Alkylthio, C₃-C₆-Alkenyl, C₃-C₆-Alkenyloxy, C₃-C₆-Alkenylthio, C₃-C₆-Alkinyl, C₃-C₆-Alkinyloxy oder C₃-C₆-Alkinylthio, C₃-C₆-Cycloalkyl, C₃-C₆-Cycloalkyloxy, C₃-C₆-Cycloalkylthio, Aryl oder Heteroaryl bedeutet, und in der
   Rⁱⁱ Wasserstoff,
   C₁-C₁₀-Alkyl, C₃-C₁₀-Alkenyl oder C₃-C₁₀-Alkinyl bedeutet, wobei diese Gruppen partiell oder vollständig halogeniert sein können und einen bis drei der folgenden Reste tragen können: Cyano, Nitro, C₁-C₄-Alkoxy, C₁-C₄-Halogenalkoxy, C₁-C₄-Alkylthio, C₁-C₄-Halogenalkylthio, C₃-C₆-Cycloalkyl, C₃-C₆-Cycloalkoxy, Aryl, Aryloxy, Heteroaryl oder Heteroaryloxy, wobei die cyclischen Reste ihrerseits partiell oder vollständig halogeniert sein können und/oder einen bis drei der folgenden Reste tragern können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino und Di-C₁-C₄-alkylamino;
und R' für eine C₁-C₃-Alkylgruppe steht.

Aus der Literatur ist bekannt, daß man 2-[2-Aryloxymethylenphenyl]-crotonsäuremethylester durch Wittig- oder Wittig-Horner analoge Umsetzung der entsprechenden α-Ketoessigsäureemthylestern erhält (EP-A 280 185, EP-A 513 580). Zur Herstellung der im wesentlichen reinen E-Isomere, die hinsichtlich ihrer biologischen Eigenschaften bevorzugt sind, sind dieser Literatur keine Angaben zu entnehmen.

Die bekannten Methoden haben den Nachteil, daß das als Nebenprodukt gebildete Triphenylphosphinoxid eine großtechnisch schwierige und aufwendige Reinigung erforderlich macht.

Demgemäß lag der vorliegenden Erfindung ein Verfahren als Aufgabe zugrunde, nach dem die im wesentlichen reinen E-Isomere der 2- [2-Aryloxymethylenphenyl]-crotonsäuremethylester erhalten werden können. Des weiteren lag der vorliegenden Erfindung ein wirtschaftliches und großtechnisch einfaches Verfahren zur Herstellung dieser Verbindungen als Aufgabe zugrunde.

Demgemäß wurde ein Verfahren zur Herstellung von im wesentlichen isomerenreinem E-2- [2-Aryloxymethylenphenyl]-crotonsäuremethylestern der Formel Ia gefunden, welches dadurch gekennzeichnet ist, daß man einen entsprechenden 2-[2-Aryloxymethylenphenyl]-α-ketoessigsäuremethylester der Formel II 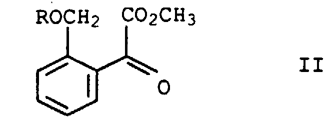 mit einer Alkyl-Metallverbindung der Formel III

M ―CH₂― R' III

in der M für ein Äquivalent eines Metallions steht, zunächst in den entsprechenden 2-[2-Aryloxymethylenphenyl]-α-hydroxybuttersäuremethylester der Formel IV 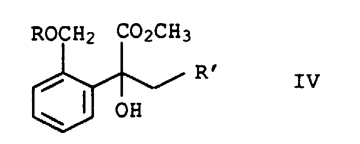 überführt, IV anschließend in Gegenwart einer Säure zu einem Gemisch aus E- und Z-2-[2-Aryloxymethylenphenyl]-crotonsäuremethylestern der Formeln Ia und Ib 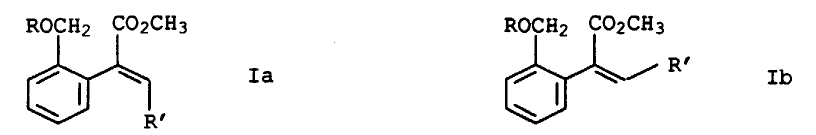 dehydratisiert, und dieses Gemisch anschließend in Gegenwart einer Base in einem inerten polaren Lösungsmittel in den im wesentlichen isomerenreinen E-2-[2-Aryloxymethylenphenyl]-crotonsäuremethylester Ia überführt.

Bei dem erfindungsgemäßen Verfahren geht man im allgemeinen so vor, daß man zunächst einen entsprechenden 2-[2-Aryloxymethylenphenyl]-α-ketoessigsäuremethylester der Formel II mit einer Alkyl-Metallverbindung der Formel III in den entsprechenden 2-[2-Aryloxymethylenphenyl]-α-hydroxybuttersäuremethylester der Formel IV überführt. 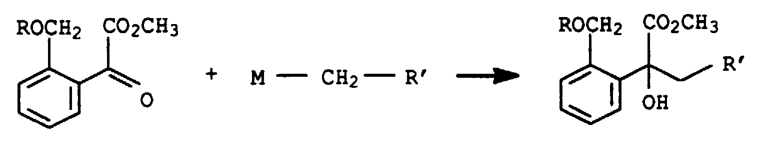 M in der Formel III bedeutet ein Äquivalent eines Metallions. Als Metallionen eignen sich beispielsweise Ionen von Übergangsmetallen oder von Erdalkalimetallen wie Magnesium, Zink, Cadmium oder Quecksilber. Besonders bevorzugt verwendet man Alkyl-magnesium-halogenide (z.B. Ethyl-magnesium Chlorid oder Ethyl-magnesium-bromid), Ethyl-zink-chlorid, Ethyl-zink-bromid, Ethyl-zink-iodid oder Diethyl-zink.

Diese Umsetzung erfolgt üblicherweise bei Temperaturen von -80°C bis 100°C, vorzugsweise -20°C bis 80°C.

Geeignete Lösungs- oder Verdünnungsmittel sind aliphatische Kohlenwasserstoffe wie Pentan, Hexan, Cyclohexan und Petrolether,aromatische Kohlenwasserstoffe wie Toluol, o-, m- und p-Xylol, Ether wie Diethylether, Diisopropylether, tert.-Butylmethylether, Dioxan, Anisol und Tetrahydrofuran, Acetale wie Formaldehyddimethylacetal und Acetondimethylketal, besonders bevorzugt Tetrahydrofuran, Diethylether, Diisopropylether, tert.-Butylmethylether, Formaldehyddimethylacetal und Acetondimethylketal. Es können auch Gemische der genannten Lösungsmittel verwendet werden.

Die Alkyl-Metallverbindungen werden im allgemeinen in mindestens äquimolaren Mengen eingesetzt. Es ist zur Steigerung der Ausbeute jedoch vorteilhaft, die Alkylverbindungen III in einem Überschuß von 500 mol-% bis 100 mol-%, vorzugsweise 300 mol-% bis 150 mol-%, insbesondere 200 mol-% bis 100 mol-%, bezogen auf die 2-[2-Aryloxymethylenphenyl]-α-ketoessigsäuremethylester der Formel II einzusetzen.

Bei der Umsetzung geht man vorteilhaft so vor, daß man die Alkyl-Metallverbindung in einem Lösungs- oder Verdünnungsmittel aufnimmt und die so erhaltene Mischung unter Kühlung portionsweise mit dem α-Ketoester II versetzt. Nach vollständiger Zugabe des Ketoesters II wird die Reaktionsmischung auf ca. 25°C (Raumtemperatur) erwärmt. Der Fortschritt der Umsetzung kann chromatografisch beobachtet werden. Nach Abschluß der Umsetzung wird die Reaktionsmischung üblicherweise zur Aufarbeitung zunächst mit einer verdünnten wäßrigen Säure oder Wasser hydrolysiert und anschließend mit einem organischen Lösungsmittel extrahiert.

Die für die Herstellung der Verbindungen I benötigten Ausgangsstoffe II sind aus der eingangs zitierten Literatur bekannt. Die Alkyl-Metallverbindungen sind ebenfalls bekannt (Houben-Weyl, Bd. 13/2, S. 53f und S. 570f) oder können gemäß der zitierten Literatur hergestellt werden.

Die so erhaltenen 2-[2-Aryloxymethylenphenyl]-α-hydroxybuttersäuremethylester der Formel IV können ohne weitere Reinigung durch Umsetzung mit einer Säure in ein Gemisch aus den E- und Z-2-[2-Aryloxymethylenphenyl]-crotonsäuremethylestern der Formeln Ia und Ib überführt werden.

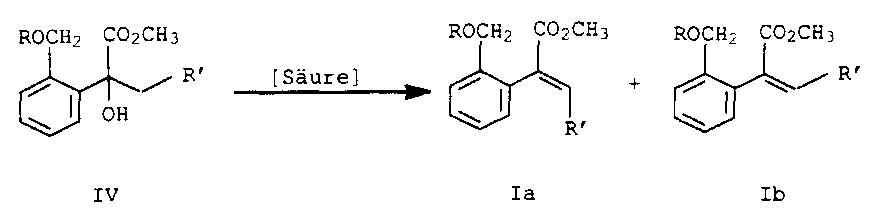

Für die Dehydratisierung eignen sich generell alle aprotischen Säuren, d.h. solche Säuren, die zur Wasserabspaltung aus α-Hydroxyestern in der Lage sind, wobei diese Säuren die Benzylether Gruppierung nicht angreifen dürfen. Als besonders vorteilhaft hierbei haben sich Säuren wie Bortrifluorid, Aluminiumtrichlorid und Eisen-III-chlorid, sowie insbesondere Säurehalogenide wie Phosgen, Thionylchlorid, Thionylbromid, Phosphortrichlorid, Phosphortribromid, Phosphoroxychlorid und Sulfonylhalogenide, erwiesen.

Die Dehydratisierung erfolgt üblicherweise bei Temperaturen von 0°C bis 230°C, vorzugsweise 20°C bis 150°C in einem inerten organischen Lösungs- oder Verdünnungsmittel unter Ausschluß von Wasser.

Geeignete Lösungs- oder Verdünnungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, besonders bevorzugt sind aromatische Kohlenwasserstoffe, Ester wie Adipinsäureester, Säureamide wie Dimethylformamid, N-Methylpyrrolidon, Tetramethylharnstoff und Dimethylpropylenharnstoff.

Es können auch Gemische der genannten Lösungsmittel verwendet werden.

Die Lewis Säuren werden im allgemeinen mindestens äquimolar, meist jedoch in einem Überschuß von 100 mol-% bis 800 mol-%, vorzugsweise 300 mol-% bis 600 mol-%, bezogen auf die 2-[2-Aryloxymethylenphenyl]-α-hydroxybuttersäuremethylester der Formel IV oder gegebenenfalls als Lösungsmittel verwendet.

Bei der Umsetzung geht man vorteilhaft so vor, daß man den α-Ketoester in Substanz oder in einem Lösungsmittel bei der Reaktionstemperatur vorlegt und anschließend die Säure zugibt. Der Fortschritt der Umsetzung kann chromatografisch beobachtet werden. Nach Abschluß der Umsetzung wird die Reaktionsmischung ohne weitere Reinigung der Isomerisierung zugeführt. Auftretende Zwischenstufen [z.B. (2-ROCH₂-C₆H₄)-C(Halogen)(CO₂CH₃)-CH₂R'] können durch erneute Behandlung mit den genannten Säuren in entsprechender Weise in Ia und Ib überführt werden.

Aus der so erhaltenen Mischung von E- und Z-2-[2-Aryloxymethylenphenyl]-crotonsäuremethylestern der Formeln Ia und Ib können die im wesentlichen isomerenreinem E-2-[2-Aryloxymethylenphenyl]-crotonsäuremethylester Ia durch Umsetzung mit einer Base erhalten werden.

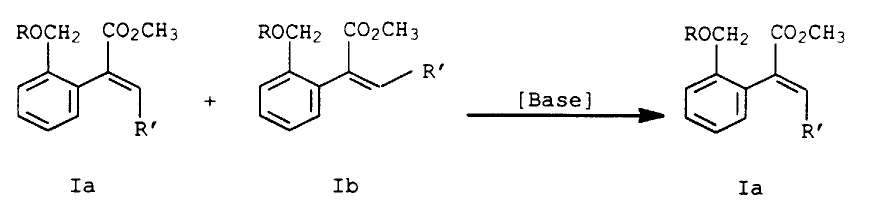

Für die Isomerisierung eignen sich Basen, wie Erdalkalimetallcarbonate wie Lithiumcarbonat und Calziumcarbonat sowie Alkalimetallhydrogencarbonate wie Natriumhydrogencarbonat, Alkalimetall- und Erdalkalimetallalkoholate wie Natriummethanolat, Natriumethanolat und Dimethoxymagnesium außerdem organische Basen, z.B. Thiophenole und Amine wie Dimethylamin, Diethylamin, Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Als besonders vorteilhaft hierbei haben sich Basen wie Natriummethanolat, Kaliummethanolat, Dimethylamin, Diethylamin, Dipropylamin, Dibutylamin, Pyridin, Natriumthiomethylat, Thiophenol und Diphenylsulfid erwiesen.

Die Isomerisierung erfolgt üblicherweise bei Temperaturen von 0°C bis 180°C, vorzugsweise 20°C bis 160°C in einem inerten organischen Lösungs- oder Verdünnungsmittel.

Geeignete Lösungs- oder Verdünnungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Toluol, Methylenchlorid, Diethylether, tert.-Butylmethylether, Tetrahydrofuran, Acetonitril, Methanol, Ethanol, Dimethylsulfoxid und Dimethylformamid.

Es können auch Gemische der genannten Lösungsmittel verwendet werden.

Die Basen werden im allgemeinen in katalytischen Mengen (z.B. 1 mol-%) bis äquimolar, vorzugsweise 1 mol-% bis 80 mol-%, insbesondere 1 mol-% bis 50 mol-%, bezogen auf die Mischung von Ia und Ib oder gegebenenfalls als Lösungsmittel verwendet.

Bei der Umsetzung geht man vorteilhaft so vor, daß man die Verbindungen Ia und Ib in einem Lösungs- oder Verdünnungsmittel aufnimmt und die so erhaltene Mischung bei 20-150°C portionsweise mit Base versetzt. Nach vollständiger Zugabe der Base wird die Reaktionsmischung bei 20-25°C oder bei der Rückflußtemperatur des Lösungsmittels belassen. Der Fortschritt der Umsetzung kann chromatografisch beobachtet werden. Nach Abschluß der Umsetzung wird die Reaktionsmischung üblicherweise vom Lösungsmittel befreit. Der Rückstand wird dann in einem nicht mit Wasser mischbaren Lösungsmittel aufgenommen (z.B. Ether und Essigsäureethylester) und mit Wasser und verdünnter wäßriger Säure gewaschen.

Nach einer weiteren Ausgestaltung des erfinderischen Verfahrens erhält man die im wesentlichen isomerenreinen E-2-[2-Aryloxymethylenphenyl]-crotonsäuremethylester Ia auch dadurch, daß man einen 2-[2-Aryloxymethylenphenyl]-a-hydroxybuttersäuremethylester der Formel IV durch Umsetzung mit einer Verbindung V in den entsprechenden Esther VI überführt und anschließend VI zu Ia eliminiert.

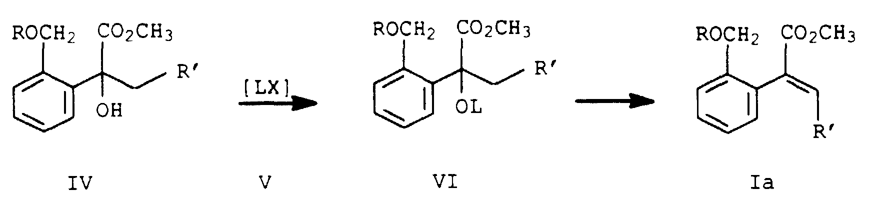

X in der Formel V steht für ein Halogenatom (z.B. Chlor, Brom oder Iod) oder eine Gruppe LO; L in den Formeln V und VI bedeutet den Rest einer Abgangsgruppe, z.B. einer aliphatischen oder aromatischen Sulfonylgruppe wie Methylsulfonyl, Trifluormethylsulfonyl, Phenylsulfonyl, 4-Methylphenylsulfonyl oder Chlorformyl.

Die Veresterung von IV mit V erfolgt in an sich bekannter Weise bei Temperaturen von 0°C bis 100°C, vorzugsweise 0°C bis 50°C in einem inerten organischen Lösungsmittel in Gegenwart einer Säure oder einer Base.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt aromatische Kohlenwasserstoffe, Ether und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat sowie Alkalimetall- und Erdalkalimetallalkoholate wie Natriummethanolat, Kaliummethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydrid, Natriummethylat, Triethylamin und Pyridin.

Die Basen werden im allgemeinen in katalytischen Mengen eingesetzt, sie können aber auch äquimolar, im Überschuß oder gegebenenfalls als Lösungsmittel verwendet werden.

Als Säuren und saure Katalysatoren finden anorganische Säuren wie Fluorwasserstoffsäure, Lewis-Säuren wie Bortrifluorid, Aluminiumtrichlorid, Eisen-III-chlorid, Zinn-IV-chlorid, Titan-IV-chlorid und Zink-II-chlorid Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, LX (V) in einem Überschuß bezogen auf IV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die anschließende Eliminierung von VI nach Ia erfolgt üblicherweise bei Temperaturen von 0°C bis 180°C, vorzugsweise 0°C bis 120°C in einem inerten organischen Lösungsmittel in Gegenwart einer Base.

Geeignete Lösungsmittel sind aliphatische Kohlenwasserstoffe wie Pentan, Hexan, Cyclohexan und Petrolether,aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril sowie Dimethylsulfoxid und Dimethylformamid, besonders bevorzugt Toluol, Xylol, Tetrahydrofuran, Acetonitril, Dimethylformamid und Dimethylsulfoxid. Es können auch Gemische der genannten Lösungsmittel verwendet werden.

Als Basen kommen bevorzugt tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Pyridin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, die Base in einem Über- oder Unterschuß bezogen auf VI einzusetzen.

Bei der Eliminierung geht man vorteilhaft so vor, daß man die Edukte in Substanz oder in einem Lösungsmittel bei 20-25°C oder unter Kühlung portionsweise mit der Base versetzt. Nach vollständiger Zugabe der Base wird die Reaktionsmischung bei der Temperatur belassen oder gegebenenfalls erwärmt. Der Fortschritt der Umsetzung kann chromatografisch beobachtet werden. Nach Abschluß der Umsetzung wird die Reaktionsmischung üblicherweise mit Wasser vermischt. Das Produkt kann aus der organischen Phase isoliert werden und gegebenenfalls durch Chromatographie weiter gereinigt werden.

Es ist außerdem möglich, die Veresterung zu VI und die Abspaltung von VI zu Ia in einem Verfahrensschritt ohne Aufarbeitung von VI durchzuführen.

Das erfindungsgemäße Verfahren eignet sich grundsätzlich zur Herstellung der aus der Literatur bekannten Verbindungen Ia. Demgemäß eignet es sich zur Herstellung von im wesentlichen isomerenreinem E-2-[2-Aryloxymethylenphenyl]-crotonsäuremethylestern der Formel Ia, in denen die Substituenten die folgende Bedeutung haben:
- R: Phenyl oder Heteroaryl, welches partiell oder vollständig halogeniert sein kann und/oder
- einen bis drei der folgenden Gruppen tragen kann: Cyano, Nitro, C₁-C₄-Alkyl, C₁-C₄-Halogenalkyl, C₁-C₄-Alkyloxy, C₁-C₄-Halogenalkyloxy, C₁-C₄-Alkylthio, C₁-C₄-Halogenalkylthio, C₃-C₈-Cycloalkyl, Phenyl, Phenoxy oder Benzyloxy, wobei die cyclischen Reste ihrerseits partiell oder vollständig halogeniert sein können und/oder einen bis drei der folgenden Gruppen tragen kann: Cyano, Nitro, C₁-C₄-Alkyl, C₁-C₄-Halogenalkyl, C₁-C₄-Alkyloxy, C₁-C₄-Halogenalkyloxy, C₁-C₄-Alkylthio, C₁-C4-Halogenalkylthio, C₃-C₈-Cycloalkyl, Phenyl, Phenoxy oder Benzyloxy, und/oder
- eine CRⁱ=NORⁱⁱ-Gruppe tragen kann, in der
   Rⁱ Wasserstoff, Cyano, Halogen, C₁-C₆-Alkyl, C₁-C₆-Alkoxy, C₁-C₆-Alkylthio, C₃-C₆-Alkenyl, C₃-C₆-Alkenyloxy, C₃-C₆-Alkenylthio, C₃-C₆-Alkinyl, C₃-C₆-Alkinyloxy oder C₃-C₆-Alkinylthio, C₃-C₆-Cycloalkyl, C₃-C₆-Cycloalkyloxy, C₃-C₆-Cycloalkylthio, Aryl oder Heteroaryl bedeutet, und in der
   Rⁱⁱ Wasserstoff,
   C₁-C₁₀-Alkyl, C₃-C₁₀-Alkenyl oder C₃-C₁₀-Alkinyl bedeutet, wobei diese Gruppen partiell oder vollständig halogeniert sein können und einen bis drei der folgenden Reste tragen können: Cyano, Nitro, C₁-C₄-Alkoxy, C₁-C₄-Halogenalkoxy, C₁-C₄-Alkylthio, C₁-C₄-Halogenalkylthio, C₃-C₆-Cycloalkyl, C₃-C₆-Cycloalkoxy, Aryl, Aryloxy, Heteroaryl oder Heteroaryloxy, wobei die cyclischen Reste ihrerseits partiell oder vollständig halogeniert sein können und/oder einen bis drei der folgenden Reste tragern können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino und Di-C₁-C₄-alkylamino;
- R': C₁-C₃-Alkyl, wie Methyl, Ethyl, Propyl oder 1-Methylethyl, insbesondere Methyl.

Die nach dem erfindungsgemäßen Verfahren erhaltenen Verbindungen Ia eignen sich zur Bekämpfung von tierischen Schädlingen oder Schadpilzen.

### Verfahrensbeispiele

### 1. Herstellung der Verbindungen der allgemeinen Formel IV:

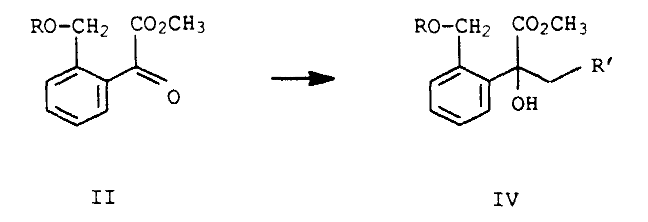

### Beispiel 1 [R = 2,5-(CH₃)₂-Phenyl, R'= CH₃]

Zu der Ethylmagnesium-Bromid-Lösung, hergestellt aus 115 g (4,72 mol) Mg-Späne und 516 g (4,72 mol) Ethylbromid in 1,1 1 Tetrahydrofuran, tropft man bei 30°C die Lösung von 300 g (2,2 mol) Zinkchlorid in 1,9 1 Tetrahydrofuran und läßt 2,5 h bei 25-30°C nachrühren. Dazu tropft man bei -20 bis -25°C und innerhalb 70 min die Lösung von II [R = 2,5-(CH₃)₂-Phenyl] in 1,25 l Tetrahydrofuran, entfernt das Kühlbad und rührt 16 h bei Raumtemperatur (RT) nach. Die Reaktionsmischung wird in eine Mischung aus 3 kg 20%iger Salzsäure und 3 kg Eis gegeben, mit 3 l MtBE (≡ Methyl-tert.-butylether) extrahiert, die Phasen getrennt und die wäßrige Phae nochmal mit 1,6 l MtBE extrahiert. Die vereinigten organischen Phasen werden nacheinander mit 2,4 l Wasser, 1,8 kg gesättigter NaHCO₃-Lösung und 1 l Wasser gewaschen und eingeengt. Man erhält 626 g IV als dickflüssiges Öl.

### 2. Herstellung der Verbindungen der allgemeinen Formel Ia und Ib

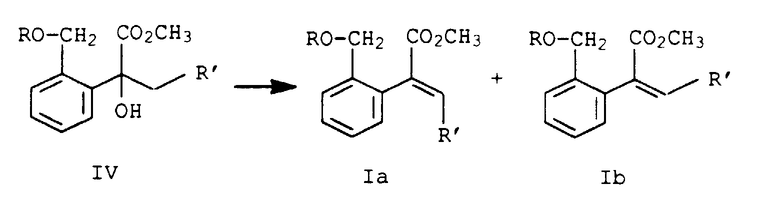

### Beispiel 2 [R = 2,5-(CH₃)₂-Phenyl, R' = CH₃]

3,8 g (11,6 mmol) der Verbindung IV aus Beispiel 1, wurden in 6,6 ml trockenem Pyridin vorgelegt. Unter Eiskühlung wurden vorsichtig 6,6 ml Phosphoroxychlorid zugetropft. Es wurde auf 105°C aufgeheizt und 1 Stunde bei dieser Temperatur nachgerührt. Nach Abkühlung auf Raumtemperatur wurde auf Eiswasser gegossen und mit Methylenchlorid extrahiert. Die vereinigten Extrakte wurden einmal mit NaHCO₃ und zweimal mit Wasser gesaschen, über Natriumsulfat getrocknet und eingeengt. Als Rohprodukt wurden 3,3 g Ia und Ib als Öl erhalten, das über Kieselgel mit Dichlorethan/Cyclohexan 1:1 als Laufmittel gereinigt wurde.
- Ia:: ¹H-NMR (CDCl₃): δ = 1,65 (d, 3H, CH₃), 2,21 (s, 3H, CH₃) 2,28 (s, 3H, CH₃), 3,79 (s, 3H, OCH₃), 4,87 (s, 2H, CH₂), 7,27 (9, 1H, CH), 6,6-7,6 (7H, ArH).
- Ib:: ¹H-NMR (CDCl₃): δ = 2,18 (d, 3H, CH₃), 2,20 (s, 3H, CH₃), 2,27 (s, 3H, CH₃), 3,86 (s, 3H, OCH₃), 4,95 (s, 2H, CH₂), 6,25 (9, 1H, CH), 6,6-7,6 (7H, ArH).

### Beispiel 3

45 g (0,14 mol) der Verbindung IV aus Beispiel 1 werden in 70 g Adipinsäuredimethylester und 0,25 g DMF (≡ Dimethylformamid) gelöst. Davon legt man 29 g vor und leitet bei einer Ölbadtemperatur von 100°C und einer Kühlertemperatur von -20°C (Kryostat) solange Phosgen ein, bis die Innentemperatur durch den Phosgen-Rückfluß bei 85°C bleibt. Bei dieser Temepratur tropft man die restliche Lösung von oben und leitet erneut Phosgen ein. Insgesamt wurden 59 g Phosgen eingeleitet. Anschließend wird 10 h bei dieser Temperatur nachgerührt und 1 h lang Stickstoff durch die Lösung geleitet. Dann gibt man 11 g Dimethylformamid und 1,25 g Eisen-III-chlorid unter Argonatmosphäre zu, rührt 15 h bei 140°C, gibt weitere 0,5 g FeCl₃ zu, und rührt weitere 4 h bei 140°C. Laut GC liegen 63,1 % Ib und 29,1 % Ia vor, die ohne weitere Reinigung der Isomerisierung zugeführt werden können.

### Beispiel 4

3,28 g der Verbindung IV aus Beispiel 1 werden in 12 g Acetonitril gelöst. Dazu gibt man 3,9 g Triethylamin und 0,43 g Lithiumchlorid. Anschließend versetzt man in kleinen Portionen mit 2,1 g p-Toluolsulfonylchlorid und rührt 1 Stunde unter Rückfluß. Nach Abkühlung wird zwischen 40 ml Methylenchlorid und 40 ml 10%iger Salzsäure verteilt, die organische Phase abgetrennt und mit 100 ml ges. NaHCO₃-Lösung gewaschen, über Na₂SO₄ getrocknet und eingeengt. Man erhält 2 g Produkt, das laut GC 53,4 % Ib und 17,5 % Ia erhält.

### 3. Herstellung der isomerenreinen Verbindungen Ia

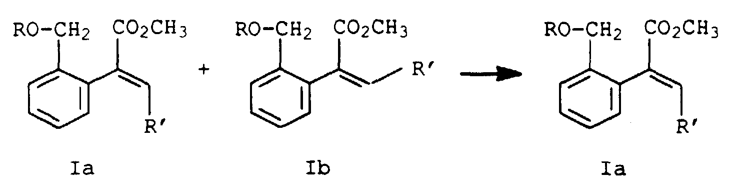

### Beispiel 5 [R = 2,5-(CH₃)₂-Phenyl, R' = CH₃]

10 g eines Gemisches aus Ia (29 %) und Ib (63,6 %) werden in 200 ml Tetrahydrofuran gelöst. Dazu gibt man 10 ml 40 %iger Dimethylamin-Lösung in Wasser, und rührt 20 h unter Rückfluß. Laut GC enthält die Reaktionslösung 93 % Ia und 3 % Ib. Zur Aufarbeitung wird eingeengt, in Essigsäureethylester aufgenommen, mit Wasser gewaschen, über Na₂SO₄ getrocknet und erneut eingeengt. Man erhält 8,8 g Ia, als farblose kristalline Verbindung. Der Gehalt an Ib-Isomer liegt unter 2 %.

### Beispiel 6 [R = 2-CH₃-Phenyl, R' = CH₃]

5 g eines Gemisches aus Ia (40 %) und Ib (55 %) werden in 100 ml Tetrahydrofuran gelöst. Dazu gibt man 10 mol-% Natriummethylat in 40 ml Methanol, und rührt 20 h unter Rückfluß. Es wird wie in Beispiel 5 aufgearbeitet. Man erhält 4 g Produkt Ia (Fp. 46°C). Der Gehalt an Ib-Isomer liegt unter 5 %.

### Beispiel 7 [R = 2-CH₃-Phenyl, R' = CH₃]

30 g (0,101 mol) eines Gemisches aus Ia (35 %) und Ib (60 %) in 500 ml Dimethylformamid werden mit 0,54 g festem Natriummethylat versetzt. Man rührt 12 h bei 130°C, läßt auf Raumtemperatur abkühlen, und fällt das Produkt durch Zugabe von Eiswasser aus. Das wird dann in Essigsäureethylester aufgenommen, mit Wasser gewaschen, über Na₂SO₄ getrocknet und eingeengt. Man erhält 28,6 g Produkt, das weniger als 5 % Ib-Isomer enthält, und beim Stehen durchkristallisiert.

## Patentansprüche

1. Verfahren zur Herstellung von im wesentlichen isomerenreinen E-2-[2-Aryloxymethylenphenyl]-crotonsäuremethylestern der Formel Ia, 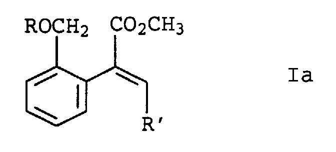 in der
R für Phenyl oder Heteroaryl, welches partiell oder vollständig halogeniert sein kann und/oder
- einen bis drei der folgenden Gruppen tragen kann: Cyano, Nitro, C₁-C₄-Alkyl, C₁-C₄-Halogenalkyl, C₁-C₄-Alkyloxy, C₁-C₄-Halogenalkyloxy, C₁-C₄-Alkylthio, C₁-C₄-Halogenalkylthio, C₃-C₈-Cycloalkyl, Phenyl, Phenoxy oder Benzyloxy, wobei die cyclischen Reste ihrerseits partiell oder vollständig halogeniert sein können und/oder einen bis drei der folgenden Gruppen tragen kann: Cyano, Nitro, C₁-C₄-Alkyl, C₁-C₄-Halogenalkyl, C₁-C₄-Alkyloxy, C₁-C₄-Halogenalkyloxy, C₁-C₄-Alkylthio, C₁-C₄-Halogenalkylthio, C₃-C₈-Cycloalkyl, Phenyl, Phenoxy oder Benzyloxy, und/oder
- eine CRⁱ=NORⁱⁱ-Gruppe tragen kann, in der
Rⁱ Wasserstoff, Cyano, Halogen,
C₁-C₆-Alkyl, C₁-C₆-Alkoxy, C₁-C₆-Alkylthio, C₃-C₆-Alkenyl, C₃-C₆-Alkenyloxy, C₃-C₆-Alkenylthio, C₃-C₆-Alkinyl, C₃-C₆-Alkinyloxy oder C₃-C₆-Alkinylthio, C₃-C₆-Cycloalkyl, C₃-C₆-Cycloalkyloxy, C3-C6-Cycloalkylthio, Aryl oder Heteroaryl bedeutet, und in der
Rⁱⁱ Wasserstoff,
C₁-C₁₀-Alkyl, C₃-C₁₀-Alkenyl oder C₃-C₁₀-Alkinyl bedeutet, wobei diese Gruppen partiell oder vollständig halogeniert sein können und einen bis drei der folgenden Reste tragen können: Cyano, Nitro, C₁-C₄-Alkoxy, C₁-C₄-Halogenalkoxy, C₁-C₄-Alkylthio, C₁-C₄-Halogenalkylthio, C₃-C₆-Cycloalkyl, C₃-C₆-Cycloalkoxy, Aryl, Aryloxy, Heteroaryl oder Heteroaryloxy, wobei die cyclischen Reste ihrerseits partiell oder vollständig halogeniert sein können und/oder einen bis drei der folgenden Reste tragern können: Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino und Di-C₁-C₄-alkylamino;
und R' für eine C₁-C₃-Alkylgruppe steht, dadurch gekennzeichnet, daß man einen entsprechenden 2-[2-Aryloxymethylenphenyl]-α-ketoessigsäuremethylester der Formel II 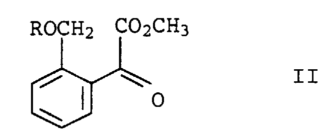 mit einer Alkyl-Metallverbindung der Formel III
M ― CH₂― R' III
in der M für ein Äquivalent eines Metallions steht, zunächst in den entsprechenden 2-[2-Aryloxymethylenphenyl]-α-hydroxybuttersäuremethylester der Formel IV 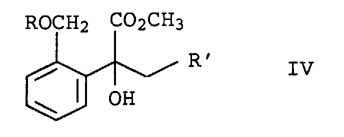 überführt, IV anschließend in Gegenwart einer Säure zu einem Gemisch aus E- und Z-2-[2-Aryloxymethylenphenyl]-crotonsäuremethylestern der Formeln Ia und Ib 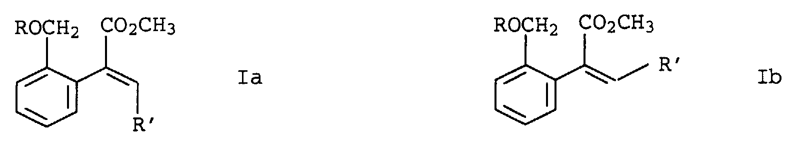 dehydratisiert, und dieses Gemisch anschließend in Gegenwart einer Base in einem inerten polaren Lösungsmittel in den im wesentlichen isomerenreinen E-2-[2-Aryloxymethylenphenyl]-crotonsäuremethylester Ia überführt.

2. Verfahren nach Anspruch 1, dadurch gekennzeichnet, daß man die Umsetzung von II mit III bei Temperaturen von -80°C bis 100°C durchführt.

3. Verfahren nach Anspruch 1, dadurch gekennzeichnet, daß man als Alkyl-Metallverbindung der Formel III ein Alkylmagnesiumhalogenid, ein Alkylzinkhalogenid oder Dialkylzink verwendet.

4. Verfahren nach Anspruch 1, dadurch gekennzeichnet, daß man als Säure Phosgen, Thionylchlorid oder Sulfonylchlorid verwendet.

5. Verfahren nach Anspruch 1, dadurch gekennzeichnet, daß man die Umsetzung von IV zum Gemisch von Ia und Ib bei Temperaturen von 20°C bis 150°C durchführt.

6. Verfahren nach Anspruch 1, dadurch gekennzeichnet, daß man die Isomerisierung des Gemisches von Ia und Ib zu im wesentlichen isomerenreinem E-2-[2-Aryloxymethylenphenyl]-crotonsäuremethylester Ia in Gegenwart eines Amins durchführt.

7. Verfahren nach Anspruch 1, dadurch gekennzeichnet, daß man die Isomerisierung des Gemisches von Ia und Ib zu im wesentlichen isomerenreinem E-2-[2-Aryloxymethylenphenyl]-crotonsäuremethylester Ia in Gegenwart eines organischen Sulfids oder Disulfids durchführt.

8. Verfahren nach Anspruch 1, dadurch gekennzeichnet, daß man die Isomerisierung des Gemisches von Ia und Ib zu im wesentlichen isomerenreinem E-2-[2-Aryloxymethylenphenyl]-crotonsäuremethylester Ia in Gegenwart eines Alkoholats durchführt.

9. 2-[2-Aryloxymethylenphenyl]-α-hydroxybuttersäuremethylester der Formel IV gemäß Anspruch 1.

10. Verwendung der 2-[2-Aryloxymethylenphenyl]-α-hydroxybuttersäuremethylester der Formel IV als Zwischenprodukte in einem Verfahren gemäß Anspruch 1.

## Claims

1. A process for the preparation of essentially isomerically pure methyl E-2-[2-aryloxymethylenephenyl]crotonates of the formula Ia 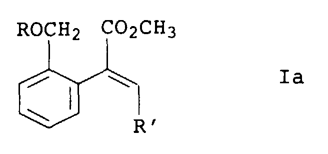 where
R is phenyl or heteroaryl which can be partially or fully halogenated, and/or
- can have attached to it from one to three of the following groups: cyano, nitro, C₁-C₄-alkyl, C₁-C₄-haloalkyl, C₁-C₄-alkyloxy, C₁-C₄-haloalkyloxy, C₁-C₄-alkylthio, C₁-C₄-haloalkylthio, C₃-C₈-cycloalkyl, phenyl, phenoxy or benzyloxy, it being possible for the cyclic radicals, in turn, to be partially or fully hydrogenated and/or to have attached to them from one to three of the following groups: cyano, nitro, C₁-C₄-alkyl, C₁-C₄-haloalkyl, C₁-C₄-alkyloxy, C₁-C₄-haloalkyloxy, C₁-C₄-alkylthio, C₁-C₄-haloalkylthio, C₃-C₈-cycloalkyl, phenyl, phenoxy or benzyloxy, and/or
- can have attached to it a CRⁱ=NORⁱⁱ group where
Rⁱ is hydrogen, cyano, halogen, C₁-C₆-alkyl, C₁-C₆-alkoxy, C₁-C₆-alkylthio, C₃-C₆-alkenyl, C₃-C₆-alkenyloxy, C₃-C₆-alkenylthio, C₃-C₆-alkynyl, C₃-C₆-alkynyloxy or C₃-C₆-alkynylthio, C₃-C₆-cycloalkyl, C₃-C₆-cycloalkyloxy, C₃-C₆-cycloalkylthio, aryl or heteroaryl and where
Rⁱⁱ is hydrogen, C₁-C₁₀-alkyl, C₃-C₁₀-alkenyl or C₃-C₁₀-alkynyl, it being possible for these groups to be partially or fully halogenated and to have attached to them from one to three of the following radicals: cyano, nitro, C₁-C₄-alkoxy, C₁-C₄-haloalkoxy, C₁-C₄-alkylthio, C₁-C₄-haloalkylthio, C₃-C₆-cycloalkyl, C₃-C₆-cycloalkoxy, aryl, aryloxy, heteroaryl or heteroaryloxy, it being possible for the cyclic radicals, in turn, to be partially or fully halogenated and/or to have attached to them from one to three of the following radicals: cyano, nitro, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylamino and di-C₁-C₄-alkylamino;
and R' is a C₁-C₃-alkyl group, which comprises first converting a suitable methyl 2-[2-aryloxymeth-ylenephenyl]-α-ketoacetate of the formula II 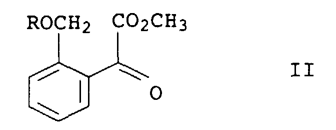 with an alkyl metal compound of the formula III
M ―CH₂― R' III
where M is a metal ion equivalent into the corresponding methyl 2-[2-aryloxymethylenephenyl]-α-hydroxybutyrate of the formula IV 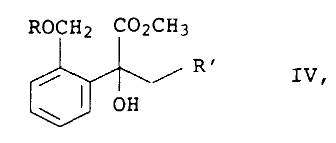 subsequently dehydrating IV in the presence of an acid to give a mixture of methyl E- and Z-2-[2-aryloxymethylenephenyl]crotonates of the formulae Ia and Ib 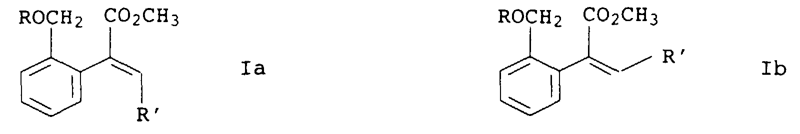 and, subsequently, converting this mixture in the presence of a base in an inert polar solvent into the essentially isomerically pure methyl E-2-[2-aryloxymethylenephenyl]crotonate Ia.

2. A process as claimed in claim 1, wherein II is reacted with III at from -80°C to 100°C.

3. A process as claimed in claim 1, wherein the alkyl metal compound of the formula III used is an alkyl magnesium halide, an alkyl tin halide or dialkylzinc.

4. A process as claimed in claim 1, wherein the acid used is phosgene, thionyl chloride or sulfonyl chloride.

5. A process as claimed in claim 1, wherein IV is reacted to give a mixture of Ia and Ib at from 20°C to 150°C.

6. A process as claimed in claim 1 wherein the mixture of Ia and Ib is isomerised to give essentially isometrically pure methyl E-2-[2-aryloxymethylenephenyl]crotonate Ia in the presence of an amine.

7. A process as claimed in claim 1, wherein the mixture of Ia and Ib is isomerized to give essentially isomerically pure methyl E-2-[2-aryloxymethylenephenyl]crotonate Ia in the presence of an organic sulfide or disulfide.

8. A process as claimed in claim 1, wherein the mixture of Ia and Ib is isomerized to give essentially isomerically pure methyl E-2-[2-aryloxymethylenephenyl]crotonate Ia in the presence of an alcoholate.

9. A methyl 2-[2-aryloxymethylenephenyl]-α-hydroxybutyrate of the formula IV as claimed in claim 1.

10. The use of the methyl 2-[2-aryloxymethylenephenyl]-α-hydroxybutyrates of the formula IV as intermediates in a process as claimed in claim 1.

## Revendications

1. Procédé pour préparer des E-2-(2-aryloxyméthylènephényl)-crotonates de méthyle à l'état d'isomères essentiellement purs, de formule Ia 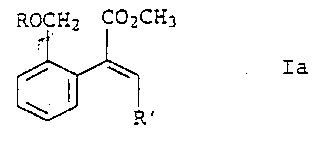 dans laquelle
R représente un groupe phényle ou hétéroaryle qui peut être partiellement ou totalement halogéné, et/ou
- qui peut porter un à trois des groupes suivants : cyano, nitro, alkyle en C1-C4, halogénoalkyle en C1-C4, alcoxy en C1-C4, halogénoalcoxy en C1-C4, alkylthio en C1-C4, halogénoalkylthio en C1-C4, cycloalkyle en C3-C8, phényle, phénoxy ou benzyloxy, les radicaux cycliques pouvant eux-mêmes être partiellement ou totalement halogénés et/ou pouvant porter un à trois des groupes suivants : cyano, nitro, alkyle en C1-C4, halogénoalkyle en C1-C4, alcoxy en C1-C4, halogénoalcoxy en C1-C4, alkylthio en C1-C4, halogénoalkylthio en C1-C4, cycloalkyle en C3-C8, phényle, phénoxy ou benzyloxy, et/ou
- qui peut porter un groupe CRⁱ=NORⁱⁱ dans lequel
Rⁱ représente l'hydrogène, un groupe cyano, un halogène, un groupe alkyle en C1-C6, alcoxy en C1-C6, alkylthio en C1-C6, alcényle en C3-C6, alcényloxy en C3-C6, alcénylthio en C3-C6, alcynyle en C3-C6, alcynyloxy en C3-C6 ou alcynylthio en C3-C6, cycloalkyle en C3-C6, cycloalcoxy en C3-C6, cycloalkylthio en C3-C6, aryle ou hétéroaryle
et dans lequel
Rⁱⁱ représente l'hydrogène,
un groupe alkyle en C1-C10, alcényle en C3-C10 ou alcynyle en C3-C10, ces groupes pouvant être partiellement ou totalement halogénés et pouvant porter un à trois des substituants suivants : cyano, nitro, alcoxy en C1-C4, halogénoalcoxy en C1-C4, alkylthio en C1-C4, halogénoalkylthio en C1-C4, cycloalkyle en C3-C6, cycloalcoxy en C3-C6, aryle, aryloxy, hétéroaryle ou hétéroaryloxy, les radicaux cycliques pouvant eux-mêmes être partiellement ou totalement halogénés et/ou pouvant porter un à trois des substituants suivants : cyano, nitro, alkyle en C1-C4, halogénoalkyle en C1-C4, alcoxy en C1-C4, halogénoalcoxy en C1-C4, alkylthio en C1-C4, halogénoalkylthio en C1-C4, alkylamino en C1-C4 et di-(alkyle en C1-C4)amino ;
et R' représente un groupe alkyle en C1-C3, caractérisé par le fait que l'on convertit d'abord un 2-(2-aryloxyméthylènephényl)-alpha-cétoacétate de méthyle correspondant de formule II 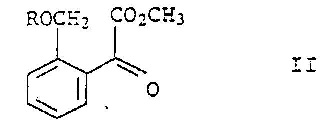
à l'aide d'un alkyl-métal de formule III
M ― CH₂ ―R' III
dans laquelle M représente un équivalent d'un ion métallique, en le 2-(2-aryloxyméthylènephényl)-alpha-hydroxybutyrate de méthyle correspondant de formule IV 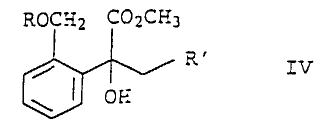
on convertit ensuite IV, par déshydratation en présence d'un acide, en un mélange des E- et Z-2-(2-aryloxyméthylènephényl)-crotonates de méthyle de formules Ia et Ib 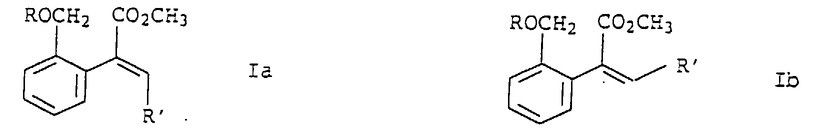
et on convertit ensuite ce mélange, en présence d'une base, dans un solvant polaire inerte, en le E-2-(2-aryloxyméthylènephényl)-crotonates de méthyle Ia à l'état d'isomère essentiellement pur.

2. Procédé selon la revendication 1, caractérisé par le fait que la réaction entre II et III est réalisée à des températures allant de -80 à 100°C.

3. Procédé selon la revendication 1, caractérisé par le fait que l'on utilise en tant qu'alkyl-métal de formule III un halogénure d'alkyl-magnésium, un halogénure d'alkyl-zinc ou un dialkyl-zinc.

4. Procédé selon la revendication 1, caractérisé par le fait que l'acide utilisé est le phosgène, le chlorure de thionyle ou le chlorure de sulfonyle.

5. Procédé selon la revendication 1, caractérisé par le fait que la conversion de IV en le mélange de Ia et Ib est réalisée à des températures de 20 à 150°C.

6. Procédé selon la revendication 1, caractérisé par le fait que l'on procède à l'isomérisation du mélange de Ia et Ib en le E-2-(2-aryloxyméthylènephényl)-crotonate de méthyle Ia à l'état d'isomère essentiellement pur en présence d'une amine.

7. Procédé selon la revendication 1, caractérisé par le fait que l'isomérisation du mélange de Ia et Ib en le E-2-(2-aryloxyméthylènephényl)-crotonate de méthyle Ia à l'état d'isomère essentiellement pur est réalisée en présence d'un sulfure ou disulfure organique.

8. Procédé selon la revendication 1, caractérisé par le fait que l'isomérisation du mélange de Ia et Ib en le E-2-(2-aryloxyméthylènephényl)-crotonate de méthyle Ia à l'état d'isomère essentiellement pur est réalisée en présence d'un alcoolate.

9. Les 2-(2-aryloxyméthylènephényl)-alpha-hydroxybutyrates de méthyle de formule IV selon la revendication 1.

10. Utilisation des 2-(2-aryloxyméthylènephényl)-alpha-hydroxybutyrates de méthyle de formule IV en tant que produits intermédiaires dans un procédé selon la revendication 1.
